# EUROPÄISCHE PATENTSCHRIFT

(11) **EP 3 960 223 B1**
(45) Veröffentlichungstag und Bekanntmachung des Hinweises auf die Patenterteilung: **04.10.2023**
(21) Anmeldenummer: 20209756.4
(22) Anmeldetag: 25.11.2020
(51) Int. Cl.: A61M 5/315, A61M 37/00

(54) **IMPLANTATSPRITZE**
IMPLANT SYRINGE
SERINGUE POUR IMPLANT

(30) Priorität: 31.08.2020 DE 102020122654
(43) Veröffentlichungstag der Anmeldung: 02.03.2022
(73) Patentinhaber: Gaplast GmbH, 82442 Altenau (DE)
(72) Erfinder: Kneer, Roland, 82490 Farchant (DE); Kneer, Stephan, 82490 Farchant (DE); Koller, Walter, 82497 Unterammergau (DE)
(74) Vertreter: Flosdorff, Jürgen

(56) Entgegenhaltungen:
- EP-A1- 3 456 374

## Beschreibung

Die Erfindung betrifft eine Implantatspritze, die dazu verwendet wird, ein strangförmiges Feststoffimplantat mit einem Langzeitwirkstoff in den Körper eines Patienten abzugeben. Meist wird das Langzeitpräparat in die Bauchdecke eines Patienten gelegt, in die zuvor mittels einer Kanüle ein Aufnahmekanal für das Feststoffimplantat eingestochen wurde.

Die Erfindung geht nach dem Oberbegriff des Patentanspruchs 1 von einer Implantatspritze aus, die folgendes Merkmale enthält: Eine Kanüle, ein Depot-Element zur Aufnahme eines Feststoffimplantats, eine Außenhülse mit einem radial überstehenden vorderen Griffabschnitt, mit dem die Kanüle gemeinsam mit dem Depot-Element und der Außenhülse axial bewegbar ist, ferner eine Innenhülse, auf der die Außenhülse sitzt und in der die Kanüle mit dem Depot-Element verschiebbar ist, wobei die Innenhülse axiale Schlitze aufweist, die radiale Stege der Außenhülse durchgreifen, wobei die Implantatspritze ferner einen stabförmigen Kolben aufweist, der an einem rückwärtigen Griffabschnitt befestigt ist und teilweise von einer hinteren Hülse umgeben ist, die verschieblich in die Innenhülse eingreifen kann, wobei der stabförmige Kolben durch das Depot-Element in die Kanüle soweit vorschiebbar ist, dass zwischen der Spitze der Kanüle und dem Kopfende des Kolbens ein Abstand verbleibt, der etwa gleich der Länge des abzugebenden Feststoffimplantats ist, wobei die Außenhülse in der vorgeschobenen Ausgangsstellung bei freiliegender Kanüle durch eine Blockiereinrichtung der Innenhülse lösbar arretiert ist und die Blockiereinrichtung beim Vorschub der hinteren Hülse in ihre Endstellung in die Freigabestellung bewegt wird, wodurch die Außenhülse mittels des vorderen Griffabschnitts auf der Innenhülse soweit zurückziehbar ist, bis sich die Kanüle innerhalb der Innenhülse befindet.

Eine derartige Implantatspritze ist in der EP 3 456 374 A1 offenbart. Bei dieser Implantatspritze ist die Außenhülse durch radiale Stege einstückig mit einer Nadelhalterung verbunden, in deren Rückseite das Depot-Element, das das Feststoffimplantat aufnimmt, eingeklebt wird. Nach der Montage von Innenhülse und Außenhülse (nachfolgend Applikator genannt) wird in eine vordere Öffnung der Nadelhalterung die Kanüle eingeklebt. Das Feststoffimplantat wird durch die offene Rückseite der Innenhülse in das Depot-Element eingesetzt und durch den stabförmigen Kolben in dem Depot-Element in die leicht eingeklemmte Ausgangsposition vorgeschoben. Bei der Anwendung der Implantatspritze zur Abgabe des Feststoffimplantats an einen Patienten wird dann der hintere Griffabschnitt mit dem Kolben vorgeschoben, bis das Feststoffimplantat bis zur Spitze der Kanüle vorgeschoben ist, woraufhin dann die in dieser Position freigegebene Außenhülse mit der Kanüle und dem Depot-Element unter Zurücklassung des Feststoffimplantats im Körper des Patienten zurückgezogen wird.

Bei der vorbekannten Implantatspritze wird die Kanüle bei fertig montiertem Applikator in die Nadelhalterung eingeklebt, die sich im Inneren der Innenhülse befindet. Dieser Vorgang ist kompliziert, und wenn er fehlerhaft verläuft, muss der gesamte Applikator verworfen werden. Außerdem ist das Einsetzen des Feststoffimplantats in die Rückseite des innerhalb der Innenhülse von der Nadelhalterung gehaltenen Depot-Elements ein komplizierter Vorgang.

Der vorliegenden Erfindung liegt die Aufgabe zugrunde, eine Implantatspritze anzugeben, bei der die Montage und die Befüllung der Implantatspritze vereinfacht sind.

Diese Aufgabe wird erfindungsgemäß durch die Merkmale des Patentanspruchs 1 gelöst.

Vorteilhafte Ausgestaltungen der Erfindung sind in den Unteransprüchen gekennzeichnet.

Die Erfindung sieht vor, dass die Kanüle mit dem Depot-Element zu einer separaten, d.h. von der übrigen Implantatspritze getrennten Kanüleneinheit fest verbunden ist, wodurch das Einsetzen eines Feststoffimplantats in das Depot-Element erheblich vereinfacht ist. Die Erfindung sieht ferner vor, dass die Stege der Außenhülse an ihren radial inneren Enden an einem ringförmigen Aufnahmeteil angesetzt sind, das sich innerhalb der Innenhülse befindet, und dass die Kanüleneinheit mit in das Depot-Element eingesetztem Feststoffimplantat in das Aufnahmeteil einsteckbar und darin arretierbar ist.

Die Kanüle wird damit außerhalb der übrigen Implantatspritze mit dem Depot-Element fest verbunden, so dass dann, wenn dieser Vorgang fehlerhaft verläuft, nur die Kanüle und das Depot-Element verworfen werden müssen. Außerdem erfolgt die Ausbildung der Kanüleneinheit unabhängig von der Herstellung der übrigen Teile der Implantatspritze und deren Montage, so dass die Herstellungszeiten und die zugehörigen Kosten der gesamten Implantatspritze erheblich verringert sind.

Mit besonderem Vorteil wird vorgeschlagen, dass die Kanüle mit dem Depot-Element umspritzt wird. Dabei versteht es sich, dass das Depot-Element ebenso wie die Außenhülse mit den Stegen und dem ringförmigen Aufnahmeteil und die Innenhülse im Spritzgussverfahren aus Kunststoff hergestellt sind.

Es liegt aber auch im Rahmen der Erfindung, dass die Kanüle in das Depot-Element eingeklebt sein kann.

Weiter wird mit Vorteil vorgeschlagen, dass das ringförmige Aufnahmeteil sich in Einschubrichtung des Depot-Elements konisch verengt, so dass das Depot-Element beim Einschub in einen leichten Klemmsitz gerät. Dabei wird der Einschub des Depot-Element in das Aufnahmeteil durch einen vorzugsweise ringförmigen Vorsprung begrenzt, der von dem Depot-Element absteht.

Weiter wird mit Vorteil vorgeschlagen, dass das Depot-Element in der Endstellung seines Einschubs durch wenigstens einen hakenförmigen Vorsprung unlösbar in dem Aufnahmeteil fixiert ist, indem der hakenförmige Vorsprung aufgrund der Elastizität des verwendeten Kunststoffs zunächst das ringförmige Aufnahmeteil aufweitet und dann hinter die rückwertige Randkante des Aufnahmeteils schnappt.

Die Kanüle der Implantatspritze ist beim Einschub des Depot-Elements in das Aufnahmeteil von einer Schutzkappe überdeckt. Hierzu schlägt die Erfindung vor, dass die Schutzkappe auf den Endabschnitt des Depot-Elements aufsetzbar ist und mit dem Depot-Element bis zu den radialen Stegen der Außenhülse in die Innenhülse einschiebbar ist. Die Schutzkappe rastet dabei bevorzugt mit einem inneren ringförmigen Vorsprung in eine umlaufende Nut des Depot-Element lösbar ein.

Weitere Merkmale und Vorteile der Erfindung ergeben sich aus der nachfolgenden Beschreibung einer bevorzugten Ausführungsform der erfindungsgemäßen Implantatspritze. Dabei zeigen:
- Figur 1: eine Aufsicht, eine Seitenansicht und einen Schnitt durch eine Kanüleneinheit;
- Figur 2: eine Seitenansicht, eine perspektivische Ansicht und eine teilweise geschnittene Ansicht einer Schutzkappe;
- Figur 3: eine Aufsicht, eine Seitenansicht und einen Schnitt durch den zusammengesetzten Zustand des Kanülenelementes mit einer Schutzkappe;
- Figur 4: eine Seitenansicht und einen Schnitt durch einen Applikator zur Bestückung mit einer befüllten Kanüleneinheit;
- Figur 5: einen vergrößerten Schnitt durch den Bereich eines konischen Aufnahmeteils mit eingesetzter Kanüleneinheit.

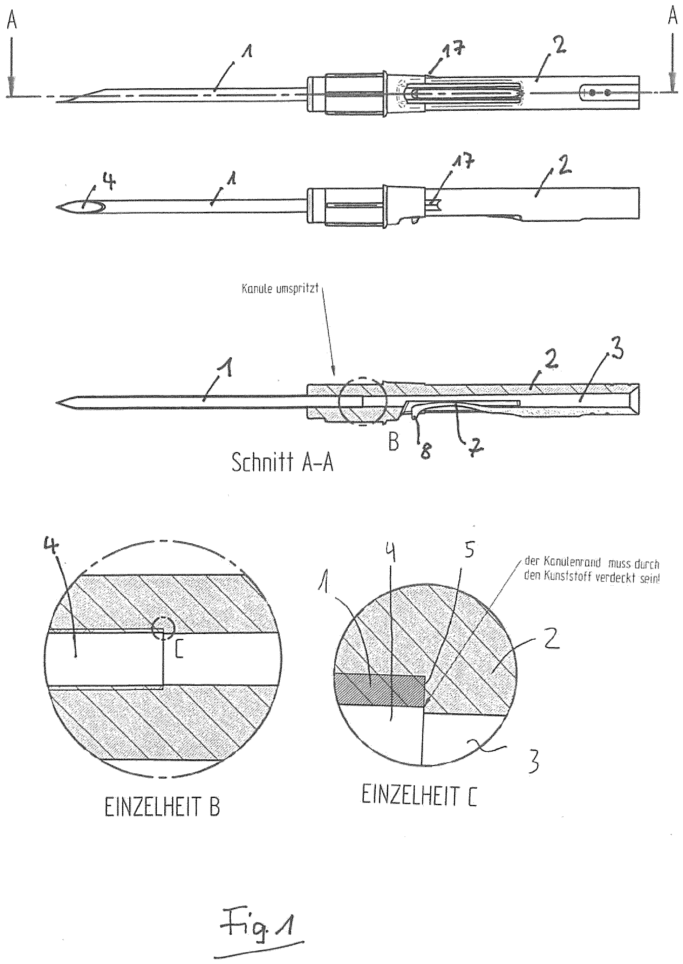

Figur 1 zeigt in mehreren Darstellungen eine Kanüle 1, die mit einem Depot-Element 2 umspritzt ist. Das Depot-Element 2 enthält einen mittigen Durchgangskanal 3, der mit dem Durchgangskanal 4 der Kanüle 1 fluchtet. Der hintere Kanülenrand 5 ist dabei durch das angespritzte Depot-Element 2 überdeckt, so dass ein in den Durchgangskanal 3 des Depot-Elements 2 eingesetztes Feststoffimplantat 6 glatt in den Durchgangskanal 4 der Kanüle 1 einschiebbar ist.

In den Durchgangskanal 3 ragt ein von der Umfangswand des Depot-Elementes 2 freigeschnittener Federarm 7, der im unbelasteten Zustand den Durchgangskanal 3 so verengt, dass ein darin aufgenommenes Feststoffimplantat 6 durch den Federarm 7 geklemmt werden kann. Der Federarm 7 hat eine leicht konvex in den Kanal 3 hineinragende Form und ist einstückig mit dem übrigen Depot-Element 2 ausgebildet. Wenn das Feststoffimplantat 6 von einem Kolben vorgeschoben wird, drückt er das Feststoffimplantat glatt durch die Engstelle, wobei der Federarm etwas nach außen gedrückt wird. Durch die Höhe der vorstehenden Nase 8 des Federarms 7 kann der Klemmdurchmesser für das Feststoffimplantat 6 angepasst werden.

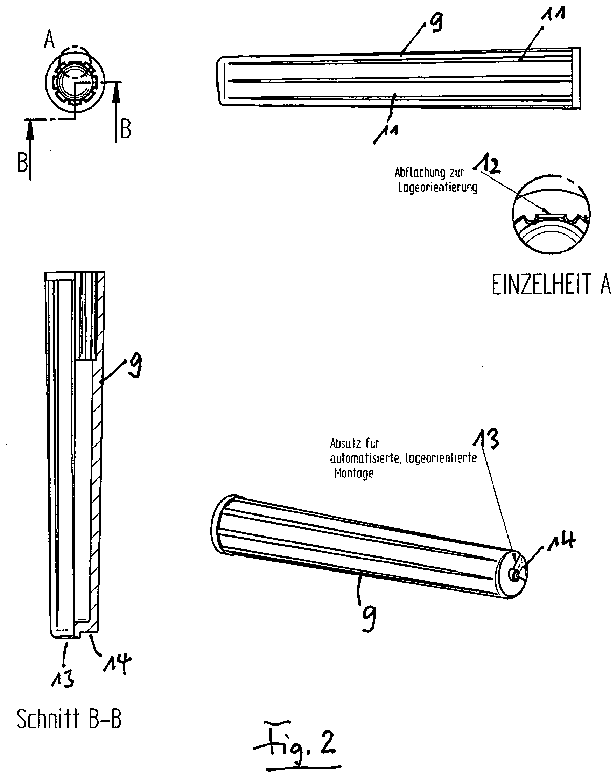

Figur 2 zeigt verschiedene Darstellungen einer Schutzkappe 9, die auf das Depot-Element 2 aufgesetzt wird, bevor dieses in den Applikator 10 eingesetzt wird. Die Schutzkappe 9 hat in Längsrichtung verlaufende Verstärkungsrippen 11, von denen eine Rippe 12 zur Lageorientierung bei automatischer Montage abgeflacht ist. Außerdem enthält die geschlossene vordere Wand 13 einen Absatz 14 für eine lageorientierte, automatisierte Montage.

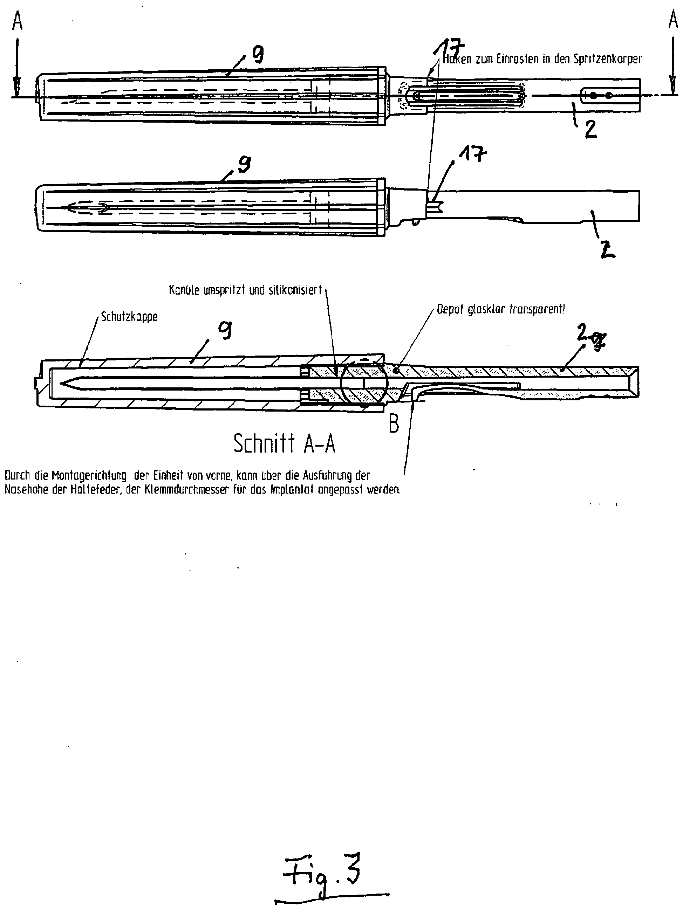

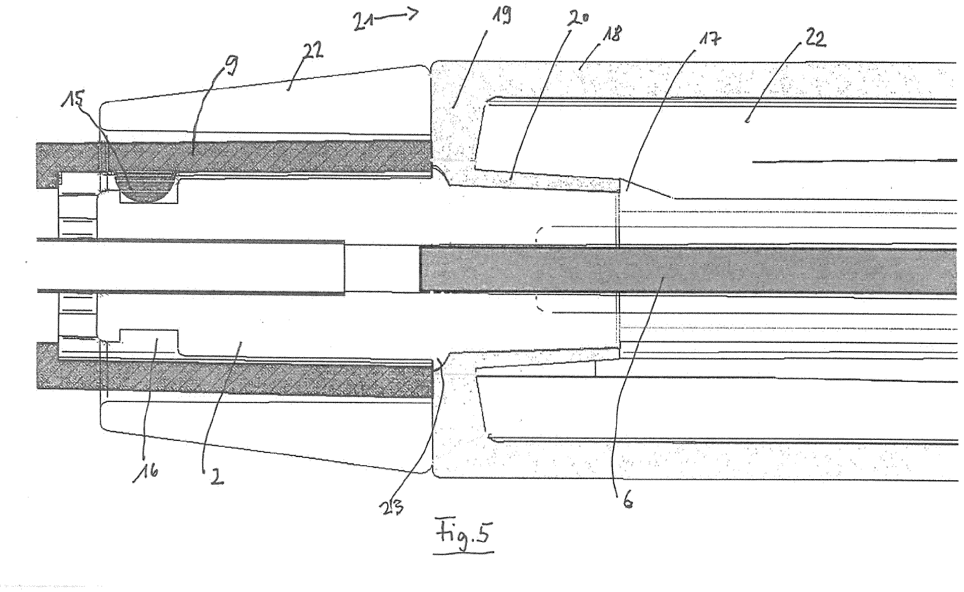

Figur 3 zeigt in verschiedenen Darstellungen den auf das Depot-Element 2 aufgesetzten Zustand der Schutzkappe 9. In der vergrößerten Darstellung der Figur 5 ist zu sehen, dass die Schutzkappe 9 mit radial inneren, noppenförmigen Vorsprüngen 15 in eine Ringnut 16 des Depot-Elements 2 einrastet, wobei die Verrastung leicht lösbar ist.

In den Figuren 1 und 3 ist ein hakenförmiger Vorsprung 17 zu erkennen, mit dem das Depot-Element 2 in dem Applikator 10 unlösbar zu verrasten ist. Figur 5 zeigt in der stark vergrößerten Darstellung, dass die Außenhülse 18 des Applikators 10 radial nach innen ragende Stege 19 hat, die einstückig mit einem ringförmigen Aufnahmeteil 20 verbunden sind. Das ringförmige Aufnahmeteil 20 verengt sich konisch in der Einschubrichtung (Pfeil 21). Die Stege 19 durchgreifen dabei in Richtung des Pfeils 21 verlaufende Schlitze in einer Innenhülse 22, auf der die Außenhülse 18 verschieblich sitzt.

Die aus Kanüle 1 und Depot-Element 2 bestehende Kanüleneinheit wird in das konische Aufnahmeteil 20 eingeschoben, wobei der hakenförmige Vorsprung 17 des Depot-Elementes 2 das ringförmige Aufnahmeteil 20 elastisch aufweitet und hinter die rückwärtige Endkante des Aufnahmteils 20 schnappt. Der Einschub wird durch einen ringförmigen Vorsprung 23 des Depot-Elements 20 begrenzt, der gemeinsam mit dem rückwärtigen Rand der Schutzkappe 9 an den radialen Stegen 19 anliegt.

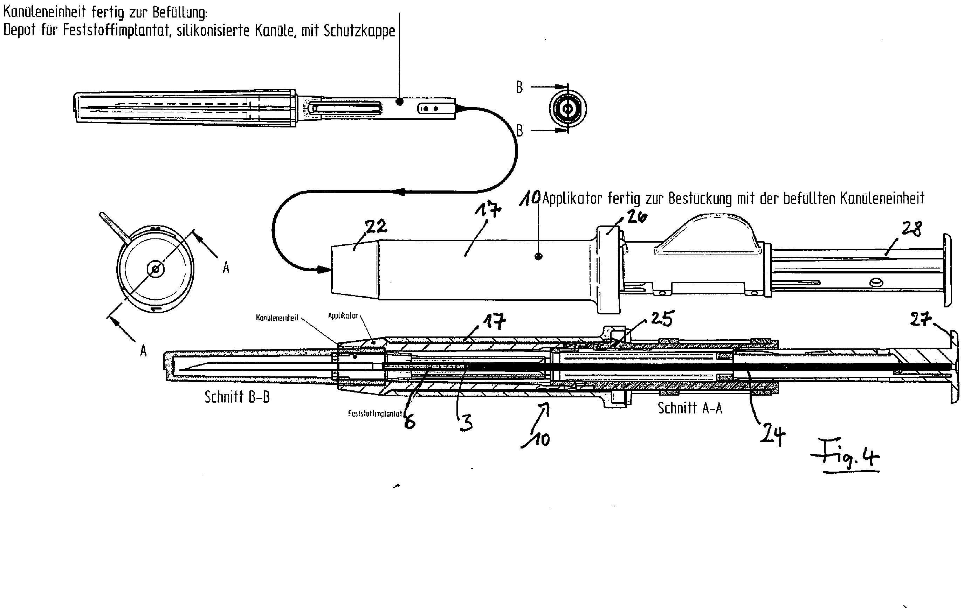

Figur 4 zeigt, dass in den Kanal 3 des Depot-Elements 2 ein rückwärtiger Kolben 24 ragt, durch den das Feststoffimplantat 6 zur Spitze der Kanüle 1 vorgeschoben werden kann. Wenn die Außenhülse 17 von einem hakenförmigen Vorsprung 25 freigegeben ist, wird sie zusammen mit der Kanüleneinheit zurückgezogen, bis sich die Kanüle 1 innerhalb der inneren Hülse 22 befindet, wobei das Feststoffimplantat 6 im Körper eines Patienten zurückbleibt.

## Patentansprüche

1. Implantatspritze mit einer Kanüle (1), mit einem Depot-Element (2) zur Aufnahme eines Feststoffimplantats (6), mit einer Außenhülse (18) mit einem radial überstehenden vorderen Griffabschnitt (26), mit dem die Kanüle (1), das Depot-Element (2) und die Außenhülse (18) gemeinsam axial bewegbar sind,
mit einer Innenhülse (22), auf der die Außenhülse (18) sitzt und in der die Kanüle (1) mit dem Depot-Element (2) verschiebbar ist, wobei die Innenhülse (22) axiale Schlitze aufweist, die radiale Stege (19) der Außenhülse (18) durchgreifen,
ferner mit einem stabförmigen Kolben (24), der an einem rückwärtigen Griffabschnitt (27) befestigt ist und teilweise von einer hinteren Hülse (28) umgeben ist, die verschieblich in die Innenhülse (22) eingreifen kann, wobei der stabförmige Kolben (24) durch das Depot-Element (2) in die Kanüle (1) so weit vorschiebbar ist, dass zwischen der Spitze der Kanüle (1) und dem Kopfende des Kolbens (24) ein Abstand verbleibt, der etwa gleich der Länge des abzugebenden Feststoffimplantats (6) ist,
wobei die Außenhülse (18) in der vorgeschobenen Ausgangsstellung bei freiliegender Kanüle (1) durch eine Blockiereinrichtung (25) der Innenhülse (22) lösbar arretiert ist und die Blockiereinrichtung (25.) beim Vorschub der hinteren Hülse (28) in ihre Endstellung in die Freigabestellung bewegt wird, wodurch die Außenhülse (18) mittels des vorderen Griffabschnitts (26) auf der Innenhülse (22) zurückziehbar ist, bis sich die Kanüle (1) innerhalb der Innenhülse (22) befindet,
**dadurch gekennzeichnet,**
**dass** die Kanüle (1) mit dem Depot-Element (2) zu einer Kanüleneinheit fest verbunden ist,
**dass** die Stege (19) der Außenhülse (18) an ihren radial inneren Enden an einem ringförmigen Aufnahmeteil innerhalb der Innenhülse (22) angesetzt sind,
und **dass** die Kanüleneinheit (1, 2) in das Aufnahmeteil (20) einsteckbar und darin arretierbar ist, wobei ein Feststoffimplantat (6) in das Depot-Element (2) eingesetzt sein kann.

2. Implantatspritze nach Anspruch 1,
**dadurch gekennzeichnet,**
**dass** die Kanüle (1) mit dem Depot-Element (2) umspritzt ist.

3. Implantatspritze nach Anspruch 1,
**dadurch gekennzeichnet,**
**dass** die Kanüle in das Depot-Element eingeklebt ist.

4. Implantatspritze nach einem der Ansprüche 1 bis 3,
**dadurch gekennzeichnet,**
**dass** das ringförmige Aufnahmeteil (20) sich in Einschubrichtung (21) des Depot-Elements (2) konisch verengt.

5. Implantatspritze nach einem der Ansprüche 1 bis 4,
**dadurch gekennzeichnet,**
**dass** der Einschub des Depot-Elements (2) in das Aufnahmeteil (20) durch einen Vorsprung (23) an dem Depot-Element (2) begrenzt ist.

6. Implantatspritze nach einem der Ansprüche 1 bis 5,
**dadurch gekennzeichnet,**
**dass** das Depot-Element (2) in der Endstellung seines Einschubs durch wenigstens einen hakenförmigen Vorsprung (17) unlösbar fixiert ist.

7. Implantatspritze nach einem der Ansprüche 1 bis 6,
**dadurch gekennzeichnet,**
**dass** eine die Kanüle (1) überdeckende Schutzkappe (9) auf den Endabschnitt des Depot-Elements (2) aufsetzbar ist, die mit dem Depot-Element (2) bis zu den radialen Stegen (19) der Außenhülse (18) in die Innenhülse (22) einschiebbar ist.

8. Implantatspritze nach Anspruch 7,
**dadurch gekennzeichnet,**
**dass** die Schutzkappe (9) lösbar in das Depot-Element (2) einrastet.

9. Implantatspritze nach einem der Ansprüche 1 bis 8,
**dadurch gekennzeichnet,**
**dass** das Depot-Element (2) aus einem durchsichtigen Kunststoff besteht.

## Claims

1. An implant syringe with a cannula (1), a storage element (2) for accommodating a solid material implant (6), an outer sleeve (18) with a radially projecting front gripping section (26), with which the cannula (1), the storage element (2) and the outer sleeve (18) are axially movable together, an inner sleeve (22), on which the outer sleeve (18) sits and in which the cannula is slidable with the storage element (2), wherein the inner sleeve (22) has axial slots, through which radial webs (19) on the outer sleeve (18) engage, further including a rod-shaped piston (24), which is fastened to a rear gripping section (27) and is partially surrounded by a rear sleeve (28), which can slidably engage in the inner sleeve (22), wherein the rod-shaped piston may be advanced through the storage element (2) into the cannula (1) so far that a gap remains between the tip of the cannula (1) and the head end of the piston (24), which is approximately equal to the length of the solid material implant (6) to be delivered, wherein the outer sleeve (18) is releasably locked in the advanced starting position with the cannula (1) exposed by a blocking device (25) and the blocking device (25) is moved into the released position when the rear sleeve (28) is advanced into its end position, whereby the outer sleeve (18) is retractable by means of the front gripping section (26) on the inner sleeve (22) until the cannula (1) is located within the inner sleeve (22), **characterised in that** the cannula (1) is firmly connected to the storage element (2) to form a cannula unit, that the webs (19) on the outer sleeve (18) are attached at their radially inner ends to an annular mounting member within the inner sleeve (22) and that the cannula unit (1, 2) is pluggable into the mounting member (20) and is lockable therein, whereby a solid material implant can be inserted into the storage element (2).

2. An implant syringe as claimed in Claim 1, **characterised in that** the cannula (1) is encapsulated with the storage element (2).

3. An impiant syringe as claimed in Claim 1, **characterised in that** the cannula is secured in the storage element by adhesive.

4. An implant syringe as claimed in one of Claims 1 to 3, **characterised in that** the annular mounting member (20) tapers conically in the insertion direction (21) of the storage element (2).

5. An implant syringe as claimed in one of Claims 1 to 4, **characterised in that** the insertion of the storage element (2) into the mounting member (20) is limited by a projection (23) on the storage element (2).

6. An implant syringe as claimed in one of Claims 1 to 5, **characterised in that** the storage element (2) is unreleasably fixed in the end position of its insertion by at least one hook-shaped projection (17),

7. An implant syringe as claimed in one of Claims 1 to 6, **characterised in that** a protective cap (9) covering the cannula (1) may be placed on the end section of the storage element (2), which may be slid with the storage element (2) into the inner sleeve (22) up to the radial webs (19) on the outer sleeve (18).

8. An implant syringe as claimed in Claim 7, **characterised in that** the protective cap (9) locks releasably in the storage element (2).

9. An implant syringe as claimed in one of Claims 1 to 8, **characterised in that** the storage element (2) consists of a transparent plastic material.

## Revendications

1. Seringue pour implant avec une canule (1), avec un élément de dépôt (2) destiné à recevoir un implant en matière solide (6), avec un manchon extérieur (18) avec une section de préhension avant (26) dépassant radialement, avec laquelle la canule (1), l'élément de dépôt (2) et le manchon extérieur (18) peuvent être déplacés axialement conjointement,
avec un manchon intérieur (22), sur lequel le manchon extérieur (18) repose et dans lequel la canule (1) peut être coulissée avec l'élément de dépôt (2), dans laquelle le manchon intérieur (22) présente des entailles axiales, qui traversent des nervures radiales (19) du manchon extérieur (18),
en outre avec un piston (24) en forme de tige, qui est fixé sur une section de préhension arrière (27) et est entouré en partie par un manchon arrière (28), qui peut venir en prise de manière à pouvoir coulisser avec le manchon intérieur (22),
dans laquelle le piston (24) en forme de tige peut être avancé si loin à travers l'élément de dépôt (2) dans la canule (1) qu'il reste un espace entre la pointe de la canule (1) et l'extrémité de tête du piston (24), lequel est à peu près égal à la longueur de l'implant en matière solide (6) à distribuer,
dans laquelle le manchon extérieur (18) est arrêté de manière amovible dans la position de départ avancée lorsque la canule (1) est à découvert par un système de blocage (25) du manchon intérieur (22) et le système de blocage (25) est déplacé dans la position de déblocage lors de l'avancement du manchon arrière (28) dans sa position finale, ce qui permet de rétracter le manchon extérieur (18) sur le manchon intérieur (22) au moyen de la section de préhension avant (26) jusqu'à ce que la canule (1) se trouve à l'intérieur du manchon intérieur (22),
**caractérisée en ce**
**que** la canule (1) est reliée de manière solidaire à l'élément de dépôt (2) en une unité à canule,
**que** les nervures (19) du manchon extérieur (18) sont placées sur leurs extrémités radialement intérieures sur une partie de réception annulaire à l'intérieur du manchon intérieur (22),
et **que** l'unité à canule (1, 2) peut être enfichée dans la partie de réception (20) et peut y être arrêtée, dans laquelle un implant en matière solide (6) peut être inséré dans l'élément de dépôt (2).

2. Seringue pour implant selon la revendication 1,
**caractérisée en ce**
**que** la canule (1) est surmoulée avec l'élément de dépôt (2).

3. Seringue pour implant selon la revendication 1,
**caractérisée en ce**
**que** la canule est collée dans l'élément de dépôt.

4. Seringue pour implant selon l'une quelconque des revendications 1 à 3,
**caractérisée en ce**
**que** la partie de réception (20) annulaire se rétrécit de manière conique dans la direction d'insertion par glissement (21) de l'élément de dépôt (2) .

5. Seringue pour implant selon l'une quelconque des revendications 1 à 4,
**caractérisée en ce**
**que** l'insertion par glissement de l'élément de dépôt (2) dans la partie de réception (20) est limitée par une partie faisant saillie (23) sur l'élément de dépôt (2).

6. Seringue pour implant selon l'une quelconque des revendications 1 à 5,
**caractérisée en ce**
**que** l'élément de dépôt (2) est fixé de manière inamovible dans la position finale de son insertion par glissement par au moins une partie faisant saillie (17) en forme de crochet.

7. Seringue pour implant selon l'une quelconque des revendications 1 à 6,
**caractérisée en ce**
**qu'**un capot de protection (9) recouvrant la canule (1) peut être placé sur la section d'extrémité de l'élément de dépôt (2), lequel peut être inséré par glissement dans le manchon intérieur (22) avec l'élément de dépôt (2) jusqu'aux nervures radiales (19) du manchon extérieur (18).

8. Seringue pour implant selon la revendication 7,
**caractérisée en ce**
**que** le capot de protection (9) s'enclenche de manière amovible dans l'élément de dépôt (2).

9. Seringue pour implant selon l'une quelconque des revendications 1 à 8,
**caractérisée en ce**
**que** l'élément de dépôt (2) est constitué d'une matière plastique transparente.
